# EUROPÄISCHE PATENTSCHRIFT

(11) **EP 1 001 979 B1**
(45) Veröffentlichungstag und Bekanntmachung des Hinweises auf die Patenterteilung: **15.06.2005**
(21) Anmeldenummer: 98936395.7
(22) Anmeldetag: 29.06.1998
(51) Int. Cl.: C07K 7/56, A61K 38/12

(54) **CYCLISCHE AZAPEPTIDE MIT ANGIOGENER WIRKUNG**
CYCLIC AZAPEPTIDES WITH ANGIOGENIC EFFECT
AZAPEPTIDES CYCLIQUES A ACTION ANGIOGENE

(30) Priorität: 04.07.1997 DE 19728524
(43) Veröffentlichungstag der Anmeldung: 24.05.2000
(73) Patentinhaber: MERCK PATENT GmbH, 64293 Darmstadt (DE)
(72) Erfinder: JONCZYK, Alfred, D-64295 Darmstadt (DE); GOODMAN, Simon, D-64287 Darmstadt (DE); KESSLER, Horst, D-65824 Schwalbach (DE); WERMUTH, Jochen, D-85095 Denkendorf (DE); SCHMITT, Jörg, D-85748 Garching (DE)
(86) Internationale Anmeldenummer: PCT/EP1998/003955
(87) Internationale Veröffentlichungsnummer: WO 1999/001472

(56) Entgegenhaltungen:
- EP-A- 0 578 083
- WO-A-93/24520
- WO-A-95/23811
- WO-A-97/25343
- WO-A-98/14220
- DE-A- 19 653 036
- GANTE J.: 'Azapeptides' SYNTHESIS 1989, Seiten 405 - 413

## Beschreibung

Die Erfindung betrifft Verbindungen der Formel I

Cyclo-(aArg-aGly-aAsp-aX-aY) I

worin
- aArg: Arg oder Aza-Arg,
- aGly: Aza-Gly,
- aAsp: Asp oder Aza-Asp,
- aX, aY: jeweils unabhängig voneinander einen Aminosäurerest ausgewählt aus einer Gruppe bestehend aus Ala, Asn, Asp, Arg, Cys, Gln, Glu, Gly, His, Ile, Leu, Lys, Met, Nle, Om, Phe, Phg, Pro, Ser, Thr, Tic, Trp, Tyr, Val, NH-Q-CO- oder
den entsprechenden Aza-aminosäuren,
- Q: Alkylen mit 1-6 C-Atomen,
bedeuten,
wobei in mindestens einer der in Formel I genannten Aminosäuren der C^{α}-Kohlenstoff durch Stickstoff ersetzt ist,
die genannten Aminosäuren auch zu N-Methyl-, N-Ethyl-, N-Propyl N-Benzyl- oder C_{α}-Methyl-derivaten derivatisiert sein können, und die Aminosäurereste über die α-Amino- oder Azagruppe und α-Carboxygruppen peptidartig miteinander verknüpft sind,
und sofern es sich um Reste optisch aktiver Aminosäuren und Aminosäurederivate handelt, sowohl die D- als auch die L-Formen eingeschlossen sind,
sowie deren Salze.

Bevorzugt werden die Verbindungen a) bis e)
a) Cyclo-(Arg-aza-Gly-Asp-D-Phe-Val);
b) Cyclo-(Arg-aza-Gly-Asp-Phe-D-Val);
c) Cyclo-(Arg-aza-Gly-Asp-Phe-N-Me-Val);
sowie deren Salze.

Ähnliche Verbindungen cyclischer Peptide sind z.B. aus EP 0 632 053, DE 195 38 741 oder EP 0 683 173 bekannt.

EP 0578 083 beschreibt Cyclo-Peptide zur Inhibition von Integrinrezeptoren.
Aza-Petide und deren Synthese werden in der Publikation von Gante F.; 1989; Synthesis; 405-413. vorgestellt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der α_{V}-, β₃- oder β₅-Integrin-Rezeptoren mit Ligänden hemmen, wie z. B. die Bindung von Fibrinogen an den β₃-Integrinrezeptor. Besondere Wirksamkeit zeigen die Verbindungen im Fall der Integrine α_{V}β₁, α_{V}β₃, α_{V}β₅, α_{IIb}β₃ sowie α_{V}β₆ und α_{V}β₈, insbesondere wurden potente selektive Inhibitoren des Vitronektinrezeptors α_{V}β₃ gefunden.

Diese Wirkung kann z.B. nach der Methode nachgewiesen werden, die von J.W. Smith et al. in J. Biol. Chem. 265, 12267-12271 (1990) beschrieben wird.
Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science 264, 569-71 (1994) beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T.-Hu, G. Klier und D.A. Cheresh in Cell 79, 1157-64 (1994) beschrieben.

Verbindungen der Formel I, die die Wechselwirkung von Integrinrezeptoren und Liganden, wie z. B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:

Die Verbindungen können die Bindung von Metallproteinasen an Integrine hemmen und so verhindern, daß die Zellen die enzymatische Aktivität der Proteinase nutzen können. Ein Beispiel ist in der Hemmbarkeit der Bindung von MMP-2- (matrix-Metallo-Proteinase-2-) an den Vitronektin-Rezeptor α_{V}β₃ durch ein Cyclo-RGD-Prptid zu finden, wie in P.C. Brooks et al., Cell 85, 683-693 (1996) beschrieben.

Die Verbreitung von Tumorzellen von einem lokalen Tumor in das vaskuläre System erfolgt durch die Bildung von Mikroaggregaten (Mikrothromben) durch Wechselwirkung der Tumorzellen mit Blutplättchen. Die Tumorzellen sind durch den Schutz im Mikroaggregat abgeschirmt und werden von den Zellen des Immunsystems nicht erkannt.
Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIa/IIIb-Antagonisten als wirksame Metastase-Hemmer angesehen werden.

Die Verbindungen der Formel I können als Arzneimittelwirkstoffe in der Human- und Veterinärmedizin eingesetzt werden, insbesondere zur Prophylaxe und/oder Therapie von Thrombose, myocardialem Infarkt, Arteriosklerose, Entzündungen, Apoplexie, Angina pectoris, Tumcrerkrankungen, osteolytischen Krankheiten wie Osteoporose, pathologisch angiogenen Krankheiten wie z. B. Entzündungen, ophthalmologischen Krankheiten, diabetischer Retinopathie, makularer Degeneration, Myopia. okularer Histoplasmose, rheumatischer Arthritis, Osteoarthritis, rubeotischem Glaukom, ulcerativer Colitis, Morbus Crohn, Atherosklerose, Pscriasis, Restenose nach Angioplastie, Multiplesklerose, viraler Infektion, bakterieller Infektion, Pilzinfektion, bei akutem Nierenversagen und bei der Wundheilung zur Unterstützung der Heilungsprozesse.

Die Verbindungen der Formel I können als antimikrobiell wirkende Substanzen bei Operationen eingesetzt werden, wo Biomaterialien, Implantate, Katheter oder Herzschrittmacher verwendet werden.

Dabei wirken sie antiseptisch. Die Wirksamkeit der antimikrobiellen Aktivität kann durch das von P.Valentin-Weigund et al., in Infecticn and Immunity, 2851-2855 (1988) beschriebene Verfahren nachgewiesen werden.

Da die Verbindungen der Formel I Inhibitoren der Fibrinogenbindung und damit Liganden der Fibrinogen rezeptoren auf Blutplättchen darstellen, können sie als Diagnostika zur Detektion und Lokelisierung von Thromben im vaskulären System *in vivo* verwendet werden, sofern sie beispielsweise durch einen radioaktiven oder UV-detektierbaren Rest substituiert werden.

Die Verbindungen der Formel I können als Inhibitoren der Fibrinogenbindung auch als wirksame Hilfsmittel zum Studium des Metabolismus von Blutplättchen in unterschiedlichen Aktivierungsstadien oder von intrazellulären Signalmechanismen des Fibrinogenrezeptors verwendet werden. Die detektierbare Einheit eines einzubauenden "Labels" , z.B. eine Isotopenmarkierung durch ³H, erlaubt es, nach Bindung an den Rezeptor, die genannten Mechanismen zu untersuchen.

In den Verbindungen der Formel I können die vorkommenden Aminosäuren derart modifiziert sein, daß der C^{α}-Kohlenstoff durch Stickstoff, unter Erhalt der Seitenkette, ersetzt ist. Es handelt sich dabei um sogenannte Azaaminosäuren.
Z.B. ist im nachstehenden Aza-tripeptid, bestehend aus den Aminosäuren Arginin, Glycin und Asparaginsäure der C^{α}-Kohlenstoff des Glycins durch Stickstoff ersetzt.

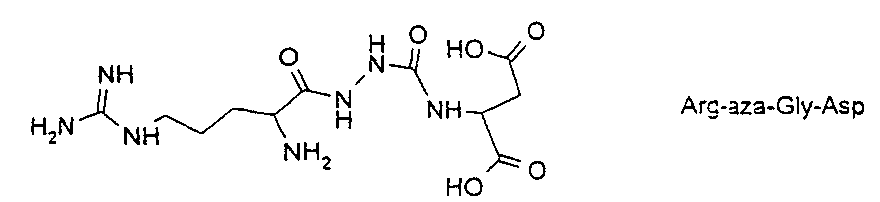

In den erfindungsgemäßen Verbindungen der Formel I liegt immer mindestens eine Aminosäure als Azaaminosäure vor.

Die vor- und nachstehend aufgeführten Abkürzungen von Aminosäureresten stehen für die Reste folgender Aminosäuren:
- Ala: Alanin
- Asn: Asparagin
- Asp: Asparaginsäure
- Arg: Arginin
- Cys: Cystein
- Gln: Glutamin
- Glu: Glutaminsäure
- Gly: Glycin
- His: Histidin
- homo-Phe: homo-Phenylalanin
- Ile: Isoleucin
- Leu: Leucin
- Lys: Lysin
- Met: Methionin
- Nle: Norleucin
- Orn: Ornithin
- Phe: Phenylalanin
- Phg: Phenylglycin
- 4-Hal-Phe: 4-Halogen-phenylalanin
- Pro: Prolin
- Sar: Sarkosin (N-Methylglycin)
- Ser: Serin
- Tic: Tetrahydroisochinolin-3-carbonsäure
- Thr: Threonin
- Trp: Tryptophan
- Tyr: Tyrosin
- Val: Valin.

Beispielhaft sind folgende Aza-aminosäuren aufgeführt:
- Aza-Arg: 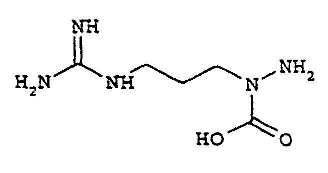
- Aza-Asp:

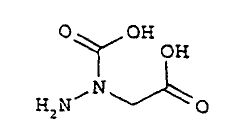

Ferner bedeuten nachstehend:
- Ac: Acetyl
- BOC: tert.-Butoxycarbonyl
- CBZ oder Z: Benzyloxycarbonyl
- DCCI: Dicyclohexylcarbodiimid
- DIPEA: Diisopropylethylamin
- DMF: Dimethylformamid
- EDCI: N-Ethyl-N,N'-(dimethylaminopropyl)-carbodiimid
- Et: Ethyl
- Fmoc: 9-Fluorenylmethoxycarbonyl
- HOBt: 1-Hydroxybenzotriazol
- Me: Methyl
- MBHA: 4-Methyl-benzhydrylamin
- Mtr: 4-Methoxy-2,3,6-trimethylphenyl-sulfonyl
- NMP: N-Methylpyrrolidon
- HONSu: N-Hydroxysuccinimid
- OBzI: Benzylester
- OtBu: tert.-Butylester
- Oct: Octanoyl
- OMe: Methylester
- OEt: Ethylester
- Pbf: 2,2,4,6,7-Pentamethyldihydrobenzofuran-5-sulfonyl-
- POA: Phenoxyacetyl
- Sal: Salicyloyl
- TBTU: O-(1H-Benzotriazol-1-yl)-N,N,N',N'-tetramethyluroniumtetrafluorborat
- TFA: Trifluoressigsäure
- Trt: Trityl (Triphenylmethyl).

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschlossen. Ferner können die Aminosäuren, z. B. als Bestandteil von Verbindungen der Formel I, mit entsprechenden an sich bekannten Schutzgruppen versehen sein.

In die erfindungsgemäßen Verbindungen sind auch sogenannte Prodrug-Derivate eingeschlossen, d. h. mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Aminosäuren, deren Konfiguration nicht speziell angegeben ist, weisen die (S)- oder (L)-Konfiguration auf.

Gegenstand der Erfindung ist ferner ein Verfahren zur Herstellung von Verbindungen der Formel I nach Anspruch 1 sowie ihrer Saize, dadurch gekennzeichnet, daß man
(a) eine Verbindung der Formel II

   H-Z-OH II

   worin
   - Z: -aArg-aGly-aAsp-aX-aY-,
   -aGly-aAsp-aX-aY-aArg-
   -aAsp-aX-aY-aArg-aGly-,
   -aX-aY-aArg-aGly-aAsp- oder
   -aY-aArg-aGly-aAsp-aX- bedeutet,
   und aArg, aGly, aAsp, aX und aY die in Anspruch 1 angegebenen Bedeutungen haben,
   oder ein reaktionsfähiges Derivat einer Verbindung der Formel 11 mit einem cyclisierenden Mittel behandelt,
   oder
b) eine Verbindung der Formel I aus einem ihrer funktionellen Derivate durch Behandeln mit einem solvolysierenden oder hydrogenolysierenden Mittel in Freiheit setzt,
   und/oder daß man eine basische oder saure Verbindung der Formel I durch Behandeln mit einer Säure oder Base in eines ihrer Salze überführt.

Vor- und nachstehend haben die Reste aArg, aGly, aAsp, aX und aY die bei den Formeln I und II angegebenen Bedeutungen, sofern nicht ausdrücklich etwas anderes angegeben ist.

In den vorstehenden Formeln steht Alkyl vorzugsweise für Methyl, Ethyl, Propyl, Isopropyl, Butyl, Isobutyl, sek.-Butyl oder tert.-Butyl, ferner auch für Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Alkylen bedeutet bevorzugt Methylen, Ethylen, Propylen, Butylen, Pentylen oder Hexylen.

Die genannten Aminosäuren und Aminosäurereste können auch zu N-Methyl-, N-Ethyl-, N-Propyl-, N-Benzyl- oder C_{α}-Methylderivaten derivatisiert sein.

Aminoschutzgruppe bedeutet vorzugsweise Acetyl, Propionyl, Butyryl, Phenylacetyl, Benzoyl, Toluyl, POA, Methoxycarbonyl, Ethoxycarbonyl, 2,2,2-Trichlorethoxycarbonyl, BOC, 2-Iodethoxycarbonyl, CBZ ("Carbobenzoxy"), 4-Methoxybenzyloxycarbonyl, FMOC, Mtr oder Benzyl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ic ausgedrückt werden, die der Formel I entsprechen und worin die nicht näher bezeichneten Reste die bei der Formel I angegebene Bedeutung haben, worin jedoch
in a)
   - aArg: Arg,
   - aGly: Aza-Gly,
   - aAsp: Asp,
   - aX, aY: jeweils unabhängig voneinander einen Aminosäurerest ausgewählt aus einer Gruppe bestehend aus Ala, Asn, Asp, Arg, Cys, Gln, Glu, Gly, His, Ile, Leu, Lys, Met, Phe, Pro, Ser, Thr, Trp, Tyr, Val oder
   den entsprechenden Aza-aminosäuren,
   bedeuten,
   und die genannten Aminosäuren auch derivatisiert sein können;
in b)
   - aArg: Arg,
   - aGly: Aza-Gly,
   - aAsp: Asp,
   - aX: Gly, Phe, D-Phe oder Aza-Phe und
   - aY: Gly, Val, Leu, Pro, D-Val, D-Leu oder D-Pro
   oder
   die entsprechenden Aza-aminosäuren,
   bedeuten,
   und die genannten Aminosäuren auch derivatisiert sein können;
in c)
   - aArg: Arg,
   - aGly: Aza-Gly,
   - aAsp: Asp,
   - aX: Gly, Phe oder D-Phe und
   - aY: Gly, N-Benzyl-Gly, Lys, D-Lys, Val, D-Val, die entsprechenden N-Alkyl-Derivate
   oder
   die entsprechenden Aza-aminosäuren,
   bedeuten,
   und die genannten Aminosäuren auch derivatisiert sein können.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart;) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, scndern sofort weiter zu den Verbindungen der Formel I umsetzt.

Verbindungen der Formel I können vorzugsweise durch Cyclisierung von Verbindungen der Formel II unter den Bedingungen einer Peptidsynthese erhalten werden. Dabei arbeitet man zweckmäßig nach üblichen Methoden der Peptidsynthese, wie sie z.B. in Houben-Weyl, 1.c., Band 15/II, Seite 1 bis 806 (1974) beschrieben sind.

Die Reaktion gelingt vorzugsweise in Gegenwart eines Dehydratisierungsmittels, z.B. eines Carbodiimids wie DCCI oder EDCI, ferner z.B. Propanphosphonsäureanhydrid (vgl. Angew. Chem. 92, 129 (1980)), Diphenylphosphorylazid oder 2-Ethoxy-N-ethoxycarbonyl-1,2-dihydrcchinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, in Dimethylsulfoxid oder in Gegenwart dieser Lösungsmittel, bei Temperaturen zwischen etwa -10 und 40, vorzugsweise zwischen 0 und 30°. Um die intramolekulare Cyclisierung vor der intermolekularen Peptidbindung zu fördern, ist es zweckmäßig, in verdünnten Lösungen zu arbeiten.
Die Reaktionszeit liegt je nach den angewendeten Bedingungen zwischen einigen Minuten und 14 Tagen.

Anstelle von Verbindungen der Formel II können auch Derivate von Verbindungen der Formel 11, vorzugsweise eine voraktivierte Carbonsäure, oder ein Carbonsäurehalogenid, ein symmetrisches oder gemischtes Anhydrid oder ein Aktivester eingesetzt werden. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, bei Verwendung eines Carbonsäurehalogenids in Gegenwart eines säurebindenden Mittels vorzugsweise einer organischen Base wie Triethylamin, Dimethylanilin, Pyridin oder Chinolin.
Auch der Zusatz eines Alkali- oder Erdalkalimetall-hydroxids. -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Ausgangsstoffe der Formel II sind in der Regel neu. Sie können nach bekannten Methoden der Peptidsynthese hergestellt werden.
Lineare Peptide können z.B. nach Merrifield (Angew. Chem. 97, 801-812 1985) an einer festen Phase, einem quellfähigen Polystyrolharz, aufgebaut werden.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/cder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"Ophenylgruppe enthalten (worin R" eine Hydroxyschutzgruppe bedeutet).

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfembar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyloder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vcr allem Aralkoxycarbonylgruppen. Beispiele für derartige Acylgruppen sind Alkanoyl wie Acetyl, Propionyl, Butyryl; Aralkanoyl wie Phenylacetyl: Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BCC, 2-lodethoxycarbonyl; Aralkyloxycarbonyl wie CBZ ("Carbobenzoxy"), 4-Methoxybenzyloxycarbonyl, FMOC; Arylsulfonyl wie Mtr. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, femer auch Alkylgruppen. Die Natur und Größe der Hydroxyschutzgruppen ist nicht kritisch, da sie nach der gewünschten chemischen Reaktion oder Reaktionsfolge wieder entfernt werden; bevcrzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran cder Dioxan, Amide wie DMF, halogenierte Kohlenwasserstoffe wie Dichlormethan, ferner auch Alkohole wie Methanol, Ethanol oder Isopropanol. sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa = und etwa 50°, vorzugsweise arbeitet man zwischen 15 und 30° (Raumtemperatur).

Die Gruppen BOC, OBut und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Die Tritylgruppe wird zum Schutz der Aminosäuren Histidin. Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenclyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren. z.B. Ameisensäure, Essigsäure, Propionsäure, Pivalirisäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits kann eine Säure der Formel I durch Umsetzung mit einer Base in eines ihrer physiologisch unbedenklichen Metall- oder Ammoniumsalze übergeführt werden. Als Salze kommen dabei insbescndere die Natrium-, Kalium-, Magnesium-, Calcium- und Ammoniumsalze in Betracht, ferner substituierte Ammoniumsalze, z. B. die Dimethyl-, Diethyl- oder Diisopropyl-ammoniumsalze, Monoethanol-, Diethanol- oder Diisopropylammoniumsalze, Cyclohexyl-, Dicyclohexylammoniumsalze, Dibenzylethylendiammoniumsalze, weiterhin z. B. Salze mit Arginin cder Lysin.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale, topische Applikation oder für eine Applikation in Form eines Inhalation-Sprays eignen und mit den neuen Verbindungen nicht reagieren, beispielsweise Wasser, pflanzliche Öle, Benzylalkohole. Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatcren, Salze zur Beeinflussung des osmotischen Druckes, Puffersubstanzen, Farb-, Geschmacks- und /oder mehrere weitere Wirkstoffe enthalten, z. B. ein oder mehrere Vitamine.
Für die Applikation als Inhalationsspray können Sprays verwendet werden, die den Wirkstoff entweder gelöst oder suspendiert in einem Treibgas oder Treibgasgemisch (z. B. CO₂ oder Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabreicht werden.

Die Verbindungen der Formel I und ihre physiologisch unbedenklichen Salze können als Integrininhibitoren bei der Bekämpfung von Krankheiten, insbesondere von Thrombosen, Herzinfarkt, koronaren Herzerkrankungen, Arteriosklerose, Tumoren, Osteoporose, Entzündungen und Infektionen verwendet werden.

Die Verbindungen der Formel I nach Anspruch 1 und/oder ihre physiologisch unbedenklichen Salze finden auch Verwendung bei pathologischen Vorgängen, die durch Angiogenese unterhalten oder propagiert werden, insbesondere bei Tumoren oder rheumatoider Arthritis.

Dabei können die erfindungsgemäßen Substanzen in der Regel in Analogie zu anderen bekannten, im Handel befindlichen Peptiden, insbesondere aber in Analogie zu den in der US-A-4 472 305 beschriebenen Verbindungen verabreicht werden, vorzugsweise in Dosierungen zwischen etwa 0,05 und 500 mg, insbesondere zwischen 0,5 und 100 mg pro Dosierungseinheit verabreicht. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die Verbindungen der Formel I als Integrinliganden zur Herstellung von Säulen für die Affinitätschromatographie zur Reindarstellung von Integrinen verwendet werden.
Der Ligand, d.h. eine Verbindung der Formel I, wird dabei über eine Ankerfunktion, z.B. die Carboxygruppe von Asp, an einen polymeren Träger kovalent gekuppelt.

Als polymere Trägermaterialien eignen sich die an sich in der Peptidchemie bekannten polymeren festen Phasen mit vorzugsweise hydrophilen Eigenschaften, beispielsweise quervernetzte Polyzucker wie Cellulose, Sepharose oder Sephadex^{R}, Acrylamide, Polymer auf Polyethylenglykolbasis oder Tentakelpolymere^{R}.

Die Herstellung der Materialien für die Affinitätschromatographie zur Integrinreinigung erfolgt unter Bedingungen wie sie für die Kondensation von Aminosäuren üblich und an sich bekannt sind.

Die Verbindungen der Formel I enthalten ein oder mehrere chirale Zentren und können daher in racemischer oder in optisch-aktiver Fcrm vorliegen. Erhaltene Racemate können nach an sich bekannten Methcden mechanisch oder chemisch in die Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen vcn Weinsäure, Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoylphenylglycin) gefüllten Säule; als Laufmittel eignet sich z.B. ein Gemisch Hexan/Isopropanol/Acetonitril, z.B. im Volumenverhältnis 82:15:3.

Natürlich ist es auch möglich, optisch aktive Verbindungen der Formel I nach den oben beschriebenen Methoden zu erhalten, indem man Ausgangsstoffe verwendet, die bereits optisch aktiv sind.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: n-Butanol/Essigsäure/Wasser 3:1:1 (A), Chloroform/Methanol 3:1 (B)
RT = Retentionszeit (Minuten) bei HPLC in den folgenden Systemen:
Säule: Nucleosil-5-C₁₈-Säule (250 x 4; 5 µm);
Als Eluenten kamen Gradienten aus Acetonitril mit 0,9 % TFA und Wasser mit 1,1 % TFA zum Einsatz (Angaben jeweils in Volumenprozent Acetonitril)
Detektion bei 220 und 254 nm.

Die Trennung der Diastereomeren erfolgt vorzugsweise unter den angegebenen Bedingungen.

Massenspektrometrie (MS): FAB (Fast Atom Bombardment) (M+H)⁺

### Beispiel 1

Zu einer Lösung von 280 mg Fmoc-Hydrazin in 20 ml Dichlcrmethan gibt man 1 Äquivalent DIPEA und 200 mg Chlorameisensäure-p-nitrophenylester (CI-CO-Pnp) in 10 ml Dichlormethan.

2 Äquivalente des erhaltenen Fmoc-NHNH-CO-Pnp in Dichlormethan und 3 Äquivalente DIPEA werden auf 1 Äquivalent H-Asp(OtBu)-Harz gegeben 1 Stunde geschüttelt.
Nach Waschen mit Dichlormethan, DMF und erneut Dichlormethan erhält man Fmoc-NHNH-CO-Asp(OtBu)-Harz.

Die Abspaltung der Fmoc-Gruppe erfolgt mit 20 % Piperidin in DMF.

In den nächsten Schritten werden Fmoc-Arg(Pbf)-OH, Fmoc-Val-OH und Fmoc-D-Phe-OH angekuppelt, wobei die Fmoc-Gruppen vcr der nachfolgenden Kupplung jeweils mit Piperidin abgespalten werden.

Man erhält Fmoc-D-Phe-Val-Arg(Pbf)-NHNH-CO-Asp(OtBu)-Harz.
Die Abspaltung des Peptids vom Harz erfolgt mit Essigsäure/Trifluorethanol/Dichlormethan (1:1:3).
Man erhält Fmoc-D-Phe-Val-Arg(Pbf)-NHNH-CO-Asp(OtBu)-OH.

Eine Lösung von 0,1 mmol Fmoc-D-Phe-Val-Arg(Pbf)-NHNH-CO-Asp(OtBu)-OH Acetat in 50 ml NMP wird langsam zu 50 ml einer Lösung von 3 Äquivalenten TBTU, 3 Äquivalenten HOBT und 10 Äquivalenten DIPEA in 50 ml NMP getropft. Nach 2 Stunden wird das Lösungsmittel entfernt und wie üblich aufgearbeitet. Man erhält man Cyclo-(Arg-aza-Gly-Asp-D-Phe-Val), RT 12,8 Min. (20-80, 30 Min.); FAB 576.

### Analog erhält man die Verbindungen

Cyclo-(Arg-aza-Gly-Asp-Phe-D-Val), RT 9,5 Min. (20-80, 30 Min.); FAB 576;
Cyclo-(Arg-aza-Gly-Asp-D-Phe-NMe-Val), FAB 590;
Cyclo-(Arg-aza-Gly-Asp-D-Lys-Val);
Cyclo-(Arg-aza-Gly-Asp-D-Phe-Lys);
Cyclo-(Arg-aza-Gly-Asp-D-Phe-Gly);
Cyclo-(Arg-aza-Gly-Asp-D-Phe-Ala);
Cyclo-(Arg-aza-Gly-Asp-D-Phe-Phe);
Cyclo-(Arg-aza-Gly-Asp-D-Phe-Leu);
Cyclo-(Arg-aza-Gly-Asp-D-Phg-Val);
Cyclo-(Arg-aza-Gly-Asp-Phe-Gly);
Cyclo-(Arg-aza-Gly-Asp-Phe-D-Ala).

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatine-kapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I
Cyclo-(aArg-aGly-aAsp-aX-aY) I
worin
aArg Arg oder Aza-Arg,
aGly Aza-Gly,
aAsp Asp oder Aza-Asp,
aX, aY jeweils unabhängig voneinander einen Aminosäurerest ausgewählt aus einer Gruppe bestehend aus Ala, Asn, Asp, Arg, Cys, Gln, Glu, Gly, His, Ile, Leu, Lys, Met, Nle, Om, Phe, Phg, Pro, Ser, Thr, Tic, Trp, Tyr, Val, NH-Q-CO-
oder
den entsprechenden Aza-aminosäuren,
Q Alkylen mit 1-6 C-Atomen,
bedeuten,
wobei in mindestens einer der in Formel I genannten Aminosäuren der C^{α}-Kohtenstoff durch Stickstoff ersetzt ist,
die genannten Aminosäuren auch zu N-Methyl-, N-Ethyl-, N-Propyl-, N-Benzyl oder C_{α}-Methylderivaten derivatisiert sein können, und die Aminosäurereste über die α-Amino- oder Azagruppe und α-Carboxygruppen peptidartig miteinander verknüpft sind,
und sofern es sich um Reste optisch aktiver Aminosäuren und Aminosäurederivate handelt, sowohl die D- als auch die L-Formen eingeschlossen sind,
sowie deren Salze.

2. Ein Enantiomer oder ein Diastereomer einer Verbindung der Formel gemäß Anspruch 1.

3. Verbindungen der Formel I gemäß Anspruch 1
a) Cyclo-(Arg-aza-Gly-Asp-D-Phe-Val);
b) Cyclo-(Arg-aza-Gly-Asp-Phe-D-Val);
c) Cyclo-(Arg-aza-Gly-Asp-Phe-N-Me-Val);
sowie deren Salze.

4. Verfahren zur Herstellung von Verbindungen der Formel I nach Anspruch 1 sowie ihrer Salze, **dadurch gekennzeichnet, daß** man
(a) eine Verbindung der Formel II
H-Z-OH II
worin
Z -aArg-aGly-aAsp-aX-aY-,
-aGly-aAsp-aX-aY-aArg-
-aAsp-aX-aY-aArg-aGly-,
-aX-aY-aArg-aGly-aAsp- oder
-aY-aArg-aGly-aAsp-aX- bedeutet,
und aArg, aGly, aAsp, aX und aY die in Anspruch 1 angegebenen Bedeutungen haben,
oder ein reaktionsfähiges Derivat einer Verbindung der Formel II mit einem cyclisierenden Mittel behandelt,
oder
b) eine Verbindung der Formel I aus einem ihrer funktionellen Derivate durch Behandeln mit einem solvolysierenden oder hydrogenolysierenden Mittel in Freiheit setzt,
und/oder daß man eine basische oder saure Verbindung der Formel I durch Behandeln mit einer Säure oder Base in eines ihrer Salze überführt.

5. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem Träger- oder Hilfsstoff in eine geeignete Dosierungsform bringt.

6. Pharmazeutische Zubereitung, **gekennzeichnet durch** einen Gehalt an mindestens einer Verbindung der Formel I nach Anspruch 1 und/oder einem ihrer physiologisch unbedenklichen Salze.

7. Verwendung von Verbindungen der Formel I nach Anspruch 1 und/oder ihre physiologisch unbedenklichen Salze zur Herstellung eines Arzneimittels.

8. Verwendung von Verbindungen der Formel I nach Anspruch 1 und ihre physiologisch unbedenklichen Salze als Integrininhibitoren zur Herstellung eines Arzneimittels zur Bekämpfung von Thrombosen, Herzinfarkt, koronaren Herzerkrankungen, Arteriosklerose, Tumoren, Osteoporose, Entzündungen und Infektionen.

9. Verwendung von Verbindungen der Formel I nach Anspruch 1 und/oder ihre physiologisch unbedenklichen Salze zur Herstellung eines Arzneimittels zur Bekämpfung von pathologischen Vorgängen, die durch Angiogenese unterhalten oder propagiert werden.

## Claims

1. Compounds of the formula I
cyclo(aArg-aGly-aAsp-aX-aY) I
in which
aArg denotes Arg or aza-Arg,
aGly denotes aza-Gly,
aAsp denotes Asp or aza-Asp,
aX, aY each, independently of one another, denote an amino acid radical selected from a group consisting of Ala, Asn, Asp, Arg, Cys, Gln, Glu, Gly, His, Ile, Leu, Lys, Met, Nle, Orn, Phe, Phg, Pro, Ser, Thr, Tic, Trp, Tyr, Val,
NH-Q-CO-
or
the corresponding aza-amino acids,
Q denotes alkylene having 1-6 C atoms,
where the C^{α} carbon in at least one of the amino acids mentioned in the formula I has been replaced by nitrogen,
the said amino acids may also be derivatised to give N-methyl, N-ethyl, N-propyl, N-benzyl or C_{α}-methyl derivatives, and the amino acid radicals are linked to one another in a peptide-like manner via the α-amino or aza group and α-carboxyl groups,
and, if the radicals are of optically active amino acids and amino acid derivatives, both the D and the L forms are included,
and salts thereof.

2. An enantiomer or a diastereomer of a compound of the formula according to Claim 1.

3. Compounds of the formula I according to Claim 1
a) cyclo(Arg-aza-Gly-Asp-D-Phe-Val);
b) cyclo(Arg-aza-Gly-Asp-Phe-D-Val);
c) cyclo(Arg-aza-Gly-Asp-Phe-N-Me-Val);
and salts thereof.

4. Process for the preparation of compounds of the formula I according to Claim 1 and salts thereof, **characterised in that**
(a) a compound of the formula II
H-Z-OH II
in which
Z denotes -aArg-aGly-aAsp-aX-aY-,
-aGly-aAsp-aX-aY-aArg-
-aAsp-aX-aY-aArg-aGly-,
-aX-aY-aArg-aGly-aAsp- or
-aY-aArg-aGly-aAsp-aX-,
and aArg, aGly, aAsp, aX and aY have the meanings indicated in Claim 1,
or a reactive derivative of a compound of the formula II is treated with a cyclising agent,
or
(b) a compound of the formula I is liberated from one of its functional derivatives by treatment with a soivoiysing or hydrogenolysing agent,
and/or **in that** a basic or acidic compound of the formula I is converted into one of its salts by treatment with an acid or base.

5. Process for the preparation of pharmaceutical compositions, **characterised in that** a compound of the formula I according to Claim 1 and/or one of its physiologically acceptable salts is converted into a suitable dosage form together with at least one excipient or adjuvant.

6. Pharmaceutical compositions, **characterised by** a content of at least one compound of the formula I according to Claim 1 and/or one of its physiologically acceptable salts.

7. Use of compounds of the formula I according to Claim 1 and/or physiologically acceptable salts thereof for the preparation of a medicament.

8. Use of compounds of the formula I according to Claim 1 and physiologically acceptable salts thereof as integrin inhibitors for the preparation of a medicament for combating thromboses, cardiac infarction, coronary heart diseases, arteriosclerosis, tumours, osteoporosis, inflammation and infections.

9. Use of compounds of the formula I according to Claim 1 and/or physiologically acceptable salts thereof for the preparation of a medicament for combating pathological processes which are maintained or propagated by angiogenesis.

## Revendications

1. Composés de la formule I
cyclo(aArg-aGly-aAsp-aX-aY) I
dans laquelle
aArg représente Arg ou aza-Arg,
aGly représente aza-Gly,
aAsp représente Asp ou aza-Asp,
aX, aY représentent, chacun indépendamment l'un de l'autre, un radical acide aminé choisi parmi un groupe constitué par Ala, Asn, Asp, Arg, Cys, Gln, Glu, Gly, His, Ile, Leu, Lys, Met, Nle, Orn, Phe, Phg, Pro, Ser, Thr, Tic, Trp, Tyr, Val, NH-Q-CO-
ou
les acides aza-aminés correspondants,
Q représente alkylène comportant de 1 à 6 atomes de C,
où le carbone C^{α} dans au moins l'un des acides aminés mentionnés dans la formule I a été remplacé par de l'azote,
lesdits acides aminés peuvent également être dérivatisés afin d'obtenir des dérivés N-méthyle, N-éthyle, N-propyle, N-benzyle ou C_{α}-méthyle, et les radicaux d'acide aminé sont liés les uns aux autres d'une manière similaire à un peptide via le groupe α-amino ou aza et des groupes α-carboxyle,
et, si les radicaux sont d'acides aminés optiquement actifs et de dérivés d'acide aminé, les formes D et L sont toutes deux incluses,
et leurs sels.

2. Un énantiomère ou un diastéréomère d'un composé de la formule selon la revendication 1.

3. Composés de la formule I selon la revendication 1
a) cyclo(Arg-aza-Gly-Asp-D-Phe-Val);
b) cyclo(Arg-aza-Gly-Asp-Phe-D-Val);
c) cyclo(Arg-aza-Gly-Asp-Phe-N-Me-Val);
et leurs sels.

4. Procédé pour la préparation de composés de la formule I selon la revendication 1 et de leurs sels, **caractérisé en ce que**
(a) un composé de la formule II
H-Z-OH II
dans laquelle
Z représente -aArg-aGly-aAsp-aX-aY-,
-aGly-aAsp-aX-aY-aArg-
-aAsp-aX-aY-aArg-aGly-,
-aX-aY-aArg-aGly-aAsp- ou
-aY-aArg-aGly-aAsp-aX-,
et aArg, aGly, aAsp, aX et aY présentent les significations indiquées dans la revendication 1,
ou un dérivé réactif d'un composé de la formule II est traité avec un agent cyclisant,
ou
(b) un composé de la formule I est libéré à partir de l'un de ses dérivés fonctionnels par traitement avec un agent de solvolyse ou d'hydrogénolyse,
et/ou **en ce qu'**un composé basique ou acide de la formule I est converti selon l'un de ses sels par traitement avec un acide ou une base.

5. Procédé pour la préparation de compositions pharmaceutiques, **caractérisé en ce qu'**un composé de la formule I selon la revendication 1 et/ou l'un de ses sels physiologiquement acceptables sont convertis selon une forme de dosage appropriée en association avec au moins un excipient ou adjuvant.

6. Compositions pharmaceutiques, **caractérisées par** une teneur en au moins un composé de la formule I selon la revendication 1 et/ou en au moins l'un de leurs sels physiologiquement acceptables.

7. Utilisation de composés de la formule I selon la revendication 1 et/ou de leurs sels physiologiquement acceptables pour la préparation d'un médicament.

8. Utilisation de composés de la formule I selon la revendication 1 et de leurs sels physiologiquement acceptables en tant qu'inhibiteurs d'intégrine pour la préparation d'un médicament pour combattre les thromboses, l'infarctus cardiaque, les maladies coronariennes, l'artériosclérose, les tumeurs, l'ostéoporose, les inflammations et les infections.

9. Utilisation de composés de la formule I selon la revendication 1 et/ou de leurs sels physiologiquement acceptables pour la préparation d'un médicament pour combattre les processus pathologiques qui sont maintenus ou propagés par une angiogénèse.
